# EUROPEAN PATENT SPECIFICATION

(11) **EP 2 229 383 B1**
(45) Date of publication and mention of the grant of the patent: **18.01.2017**
(21) Application number: 08856804.3
(22) Date of filing: 25.11.2008
(51) Int. Cl.: C07D 413/12, C07D 413/14, A61K 31/497, A61P 25/28

(54) **ISOXAZOLO-PYRAZINE DERIVATIVES**
ISOXAZOLO-PYRAZIN-DERIVATE
DÉRIVÉS D'ISOXAZOLO-PYRAZINE

(30) Priority: 04.12.2007 EP 07122271
(43) Date of publication of application: 22.09.2010
(73) Proprietor: F. Hoffmann-La Roche AG, 4070 Basel (CH)
(72) Inventor: BUETTELMANN, Bernd, 79650 Schopfheim (DE); JAKOB-ROETNE, Roland, 79594 Inzlingen (DE); KNUST, Henner, 79618 Rheinfelden (DE); THOMAS, Andrew, CH-4102 Binningen (CH)
(74) Representative: Salud, Carlos E.
(86) International application number: PCT/EP2008/066127
(87) International publication number: WO 2009/071464

(56) References cited:
- WO-A-2006/037480
- WO-A-2007/039389

## Description

The present invention is concerned with isoxazolo-pyrazine derivatives having affinity and selectivity for GABA A α5 receptor binding site, their manufacture, pharmaceutical compositions containing them and their use as medicaments. The active compounds of the present invention are useful as cognitive enhancer or for the treatment of cognitive disorders like Alzheimer's disease.

In particular, the present invention is concerned with Isoxazolo-Pyrazine derivatives of formula I 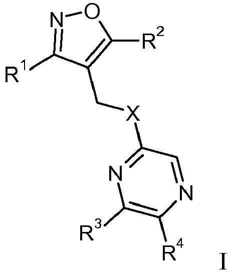 wherein R¹, R², R³, R⁴ and X are as described in claim 1.

Receptors for the major inhibitory neurotransmitter, gamma-aminobutyric acid (GABA), are divided into two main classes: (1) GABA A receptors, which are members of the ligand-gated ion channel superfamily and (2) GABA B receptors, which are members of the G-protein linked receptor family. The GABA A receptor complex which is a membrane-bound heteropentameric protein polymer is composed principally of α, β and γ subunits.

Presently a total number of 21 subunits of the GABA A receptor have been cloned and sequenced. Three types of subunits (α, β and γ) are required for the construction of recombinant GABA A receptors which most closely mimic the biochemical, electrophysiological and pharmacological functions of native GABA A receptors obtained from mammalian brain cells. There is strong evidence that the benzodiazepine binding site lies between the α and γ subunits. Among the recombinant GABA A receptors, α1β2γ2 mimics many effects of the classical type-I BzR subtypes, whereas α2β2γ2, α3β2γ2 and α5β2γ2 ion channels are termed type-II BzR.

It has been shown by McNamara and Skelton in Psychobiology, 21:101-108 that the benzodiazepine receptor inverse agonist β-CCM enhance spatial learning in the Morris watermaze. However, β-CCM and other conventional benzodiazepine receptor inverse agonists are proconvulsant or convulsant which prevents their use as cognition enhancing agents in humans. In addition, these compounds are non-selective within the GABA A receptor subunits, whereas a GABA A α5 receptor partial or full inverse agonist which is relatively free of activity at GABA A α1 and/or α2 and/or α3 receptor binding sites can be used to provide a medicament which is useful for enhancing cognition with reduced or without proconvulsant activity. It is also possible to use GABA A α5 inverse agonists which are not free of activity at GABA A α1 and/or α2 and/or α3 receptor binding sites but which are functionally selective for α5 containing subunits. However, inverse agonists which are selective for GABA A α5 subunits and are relatively free of activity at GABA A α1, α2 and α3 receptor binding sites are preferred.

WO 2006/037480 A discloses hexafluoroisopropanol substituted ether derivatives which are stated to be useful in the treatment of diseases such as Alzheimer's disease.

WO 2007/039389 A discloses aryl-isoxazole-4-carbonyl-pyrrole-2-carboxylic acid amide derivatives which might be useful as cognitive enhancer or for the treatment of cognitive disorders like Alzheimer's disease.

Objects of the present invention are compounds of formula I and pharmaceutically acceptable salts, the preparation of the above mentioned compounds, medicaments containing them and their manufacture as well as the use of the above mentioned compounds in the control or prevention of illnesses, especially of illnesses and disorders of the kind referred to earlier or in the manufacture of corresponding medicaments.

The most preferred indication in accordance with the present invention is Alzheimer's disease

The following definitions of the general terms used in the present description apply irrespective of whether the terms in question appear alone or in combination.

As used herein, the term "alkyl" denotes a saturated straight- or branched-chain group containing from 1 to 7 carbon atoms, for example, methyl, ethyl, propyl, isopropyl, n-butyl, *iso-*butyl, *sec*-butyl, *tert*-butyl and the like. Preferred alkyl groups are groups with 1 to 4 carbon atoms.

The term "halo-C₁₋₇-alkyl", "C₁₋₇-haloalkyl"or "C₁₋₇-alkyl optionally substituted with halo" denotes a C₁₋₇-alkyl group as defined above wherein at least one of the hydrogen atoms of the alkyl group is replaced by a halogen atom, preferably fluoro or chloro, most preferably fluoro. Examples of halo-C₁₋₇-alkyl include but are not limited to methyl, ethyl, propyl, isopropyl, isobutyl, sec-butyl, tert-butyl, pentyl or n-hexyl substituted by one or more Cl, F, Br or I atom(s), in particular one, two or three fluoro or chloro, as well as those groups specifically illustrated by the examples herein below. Among the preferred halo-C₁₋₇-alkyl groups are difluoro- or trifluoro-methyl or -ethyl.

The term "hydroxy-C₁₋₇-alkyl", "C₁₋₇-hydroxyalkyl" or "C₁₋₇-alkyl optionally substituted with hydroxy" denotes a C₁₋₇-alkyl group as defined above wherein at least one of the hydrogen atoms of the alkyl group is replaced by a hydroxy group. Examples of hydroxy-C₁₋₇-alkyl include but are not limited to methyl, ethyl, propyl, isopropyl, isobutyl, sec-butyl, tert-butyl, pentyl or n-hexyl substituted by one or more hydroxy group(s), in particular with one, two or three hydroxy groups, preferably with one hydroxy group, as well as those groups specifically illustrated by the examples herein below.

The term "cyano-C₁₋₇-alkyl", "C₁₋₇-cyanoalkyl" or "C₁₋₇-alkyl optionally substituted with cyano" denotes a C₁₋₇-alkyl group as defined above wherein at least one of the hydrogen atoms of the alkyl group is replaced by a cyano group. Examples of hydroxy-C₁₋₇-alkyl include but are not limited to methyl, ethyl, propyl, isopropyl, isobutyl, sec-butyl, tert-butyl, pentyl or n-hexyl substituted by one or more cyano group(s), preferably by one, two or three, and more preferably by one cyano group, as well as those groups specifically illustrated by the examples herein below.

The term "alkoxy" denotes a group -O-R wherein R is alkyl as defined above.

The term "aryl" refers to a monovalent aromatic carbocyclic ring system, preferably to phenyl or naphthyl, and more preferably to phenyl. Aryl is optionally substituted as described herein.

The term "aromatic" means aromatic according to Hückel's rule. A cyclic molecule follows Hückel's rule when the number of its π-electrons equals 4*n* + 2 where n is zero or any positive integer.

The term "halo" or "halogen" denotes chloro, iodo, fluoro and bromo.

The term "C₁₋₇-haloalkoxy" or "halo-C₁₋₇-alkoxy" denotes a C₁₋₇-alkoxy group as defined above wherein at least one of the hydrogen atoms of the alkoxy group is replaced by a halogen atom, preferably fluoro or chloro, most preferably fluoro. Examples of halo-C₁₋₇-alkoxy include but are not limited to methyl, ethyl, propyl, isopropyl, isobutyl, sec-butyl, tert-butyl, pentyl or n-hexyl substituted by one or more Cl, F, Br or I atom(s), in particular one, two or three fluoro or chloro atoms, as well as those groups specifically illustrated by the examples herein below. Among the preferred halo-C₁₋₇-alkoxy groups are difluoro- or trifluoro-methoxy or -ethoxy substituted as described above, preferably -OCF₃.

The term "cycloalkyl" refers to a monovalent saturated cyclic hydrocarbon radical of 3 to 7 ring carbon atoms, preferably 3 to 6 carbon atoms, such as cyclopropyl, cyclobutyl, cyclopentyl, or cyclohexyl.
The term "heterocycloalkyl" refers to a monovalent 3 to 7 membered saturated monocyclic ring containing one, two or three ring heteroatoms selected from N, O or S. One or two ring heteroatoms are preferred. Preferred are 4 to 6 membered heterocycloalkyl or 5 to 6 membered heterocycloalkyl, each containing one or two ring heteroatoms selected from N, O or S. Examples for heterocycloclakyl moieties are tetrahydrofuranyl, tetrahydropyranyl, pyrrolidinyl, morpholinyl, thiomorpholinyl, piperidinyl, or piperazinyl. A preferred heterocycloalkyl is tetrahydropyranyl. Heterocycloalkyl is a subgroup of "heterocyclyl" as described below. Heterocycloalkyl is optionally substituted as described herein.

The term "heteroaryl" refers to a monovalent aromatic 5- or 6-membered monocyclic ring containing one, two, or three ring heteroatoms selected from N, O, or S, the remaining ring atoms being C. Preferably, the 5- or 6-membered heteroaryl ring contains one or two ring heteroatoms. 6-membered heteroaryl are preferred. Examples for heteroaryl moieties include but are not limited to thiophenyl, furanyl, pyridinyl, pyrimidinyl, pyrazinyl, pyrazolyl, imidazolyl, oxazolyl, isoxazolyl, thiazolyl, isothiazolyl, triazolyl, 1,2,4-oxadiazolyl, or 1,3,4-oxadiazolyl.

The term "heterocyclyl" or "heterocyclyl moiety" refers to a monovalent saturated or partially saturated 3- to 7-membered monocyclic or 9- to 10-membered bicyclic ring system wherein one, two, three or four ring carbon atoms have been replaced by N, O or S, and with the attachment point on the saturated or partially unsaturated ring of said ring system. Such bicyclic heterocyclyl moieties hence include aromatic rings annelated to saturated rings. Where applicable, "heterocyclyl moiety" further includes cases where two residues R' and R" together with the nitrogen to which they are bound form such a heterocyclyl moiety. Examples for heterocyclyl include but are not limited to tetrahydrofuranyl, tetrahydropyranyl, pyrrolidinyl, morpholinyl, thiomorpholinyl, piperidinyl, piperaziny, or hexahydrothiopyranyl, as well as their corresponding partially unsaturated derivatives.

The term "oxo" when referring to substituents on heterocycloalkyl, heterocyclyl or on a heterocycle means that an oxygen atom is attached to the ring. Thereby, the "oxo" may either replace two hydrogen atoms on a carbon atom, or it may simply be attached to sulfur, so that the sulfur exists in oxidized form, i.e. bearing one or two oxygens.

When indicating the number of subsituents, the term "one or more" means from one substituent to the highest possible number of substitution, i.e. replacement of one hydrogen up to replacement of all hydrogens by substituents. Thereby, one, two or three substituents are preferred.

The term "pharmaceutically acceptable salt" or "pharmaceutically acceptable acid addition salt" embraces salts with inorganic and organic acids, such as hydrochloric acid, nitric acid, sulfuric acid, phosphoric acid, citric acid, formic acid, fumaric acid, maleic acid, acetic acid, succinic acid, tartaric acid, methane-sulfonic acid, p-toluenesulfonic acid and the like.

In detail, the present invention relates to compounds of the general formula (I) 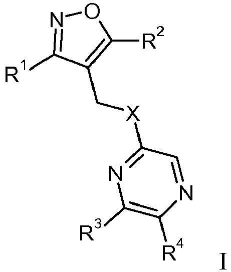 wherein
X is O or NH;
R¹ is phenyl or pyridine-2-yl, optionally substituted with one, two or three halo,
R² is C₁₋₄alkyl or H;
R³ and R⁴ each are independently
   H,
   C₁₋₇alkyl, optionally substituted with one or more halo, cyano, or hydroxy,
   C₁₋₇alkoxy, optionally substituted with one or more halo,
   CN,
   halo,
   NO₂,
   -C(O)-R^{a}, wherein R^{a} is hydroxy, C₁₋₇alkoxy, C₁₋₇alkyl, phenoxy or phenyl,
   -C(O)-NR^{b}R^{c}, wherein R^{b} and R^{c} are each independently
      H, C₁₋₇alkyl, optionally substituted with one or more halo, hydroxy, or cyano,
      -(CH₂)_{z}-C₃₋₇cycloalkyl, optionally substituted by one or more B, and z is 0, 1, 2, 3 or 4,
      -(CH₂)_{y}-heterocyclyl, wherein y is 0, 1, 2, 3 or 4, and wherein heterocyclyl is optionally substituted by one or more A
      R^{b} and R^{c} together with the nitrogen to which they are bound form a heterocyclyl moiety , optionally substituted with one or more A, or
   or R³ and R⁴ together form an annelated benzo ring, the benzo ring is optionally substituted by one or more E,
A is hydroxy, oxo, C₁₋₇alkyl, C₁₋₇alkoxy, C₁₋₇haloalkyl, C₁₋₇hydroxyalkyl, halo, or CN,
B is halo, hydroxy, CN, C₁₋₄alkyl, or C₁₋₄haloalkyl,
E is halo, CN, NO₂, hydroxy, C₁₋₇alkyl, C₁₋₇alkoxy, C₁₋₇haloalkyl, C₁₋₇hydroxyalkyl, C₁₋₇cyanoalkyl, C₁₋₇haloalkoxy, or C₃₋₇cycloalkyl,
or a pharmaceutically acceptable salt thereof

In certain embodiments of the compound of formula I, X is O or NH. Each of these alternatives may be combined with any other embodiment as disclosed herein.

Further, it is to be understood that every embodiment relating to a specific residue R¹ to R⁴ as disclosed herein may be combined with any other embodiment relating to another residue R¹ to R⁴ as disclosed herein.

In certain embodiments of the compound of formula I, R¹ is phenyl, optionally substituted with one, two or three halo. Preferred halo substituents are chloro and fluoro. In case phenyl is substituted, preferably one or two optional halo substituents selected from chloro or fluoro are chosen.

In certain embodiments of the invention, R² is C₁₋₄alkyl or H. Preferably, R² is methyl or H.

In certain embodiments of the compound of formula I, R³ and R⁴ are as defined above.

In certain embodiments of the compound of formula I, R³ is H or R³ and R⁴ together form an anellated benzo ring, i.e. a benzo ring anellated to the pyrazin moiety, whereby benzo is optionally substituted as defined herein.

In certain embodiments of the compound of formula I, R⁴ is as described above.

In certain embodiments of the compound of formula I, R⁴ is
H,
-C(O)-R^{a}, wherein R^{a} is hydroxy, C₁₋₇alkoxy, C₁₋₇alkyl, phenoxy or phenyl,
-C(O)-NR^{b}R^{c}, wherein R^{b} and R^{c} are each independently
H,
C₁₋₇alkyl, optionally substituted with one or more halo, hydroxy, or cyano,
-(CH₂)_{z}-C₃₋₇cycloalkyl, optionally substituted by one or more B, and z is 0, 1, 2, 3 or 4, preferably 0 or 1,
-(CH₂)_{y}-heterocyclyl, wherein y is 0, 1, 2, 3 or 4, preferably 0, and wherein heterocyclyl is optionally substituted by one or more A
or R³ and R⁴ together form an annelated benzo ring, the benzo ring is optionally substituted by one or more E, A is hydroxy, oxo, C₁₋₇alkyl, C₁₋₇alkoxy, C₁₋₇haloalkyl, C₁₋₇hydroxyalkyl, halo, or CN,
B is halo, hydroxy, CN, C₁₋₄alkyl, or C₁₋₄haloalkyl,
E is halo, CN, NO₂, hydroxy, C₁₋₇alkyl, C₁₋₇alkoxy, C₁₋₇haloalkyl, C₁₋₇hydroxyalkyl, C₁₋₇cyanoalkyl, C₁₋₇haloalkoxy, or C₃₋₇cycloalkyl.

In certain embodiments of the compound of formula I, R⁴ is
H,
-C(O)-R^{a}, wherein R^{a} is hydroxy, or C₁₋₇alkoxy,
-C(O)-NR^{b}R^{c}, wherein R^{b} and R^{c} are each independently
   H,
   C₁₋₇alkyl, optionally substituted with one or more halo, hydroxy, or cyano,
   -(CH₂)_{z}-C₃₋₇cycloalkyl, optionally substituted by one or more B, and z is 0, 1, 2, 3 or 4, preferably 0 or 1,
   -(CH₂)_{y}-heterocyclyl, wherein y is 0, 1, 2, 3 or 4, preferably 0, and wherein heterocyclyl is tetrahydropyranyl optionally substituted by one or more A
or R³ and R⁴ together form an annelated benzo ring, the benzo ring is optionally substituted by one or more E,
A is hydroxy, oxo, C₁₋₇alkyl, C₁₋₇alkoxy, C₁₋₇haloalkyl, C₁₋₇hydroxyalkyl, halo, or CN,
B is halo, hydroxy, CN, C₁₋₄alkyl, or C₁₋₄haloalkyl,
E is halo, CN, NO₂, hydroxy, C₁₋₇alkyl, C₁₋₇alkoxy, C₁₋₇haloalkyl, C₁₋₇hydroxyalkyl, C₁₋₇cyanoalkyl, C₁₋₇haloalkoxy, or C₃₋₇cycloalkyl.

A certain embodiment of the invention comprises the compound of formula I 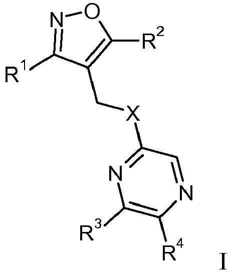 wherein
X is O or NH;
R¹ is phenyl or pyridine-2-yl" optionally substituted with one, two or three halo,
R² is C₁₋₄alkyl or H;
R³ is H;
R⁴ is H,
   - C(O)-R^{a}, wherein R^{a} is hydroxy, C₁₋₇alkoxy, C₁₋₇alkyl, phenoxy or phenyl,
   - C(O)-NR^{b}R^{c}, wherein R^{b} and R^{c} are each independently
      H,
      C₁₋₇alkyl, optionally substituted with one or more halo, hydroxy, or cyano,
      -(CH₂)_{z}-C₃₋₇cycloalkyl, optionally substituted by one or more B, and z is 0, 1, 2, 3 or 4, preferably 0 or 1,
   - (CH₂)_{y}-heterocyclyl, wherein y is 0, 1, 2, 3 or 4, preferably 0, and wherein heterocyclyl is optionally substituted by one or more A
or R³ and R⁴ together form an annelated benzo ring, the benzo ring is optionally substituted by one or more E,
A is hydroxy, oxo, C₁₋₇alkyl, C₁₋₇alkoxy, C₁₋₇haloalkyl, C₁₋₇hydroxyalkyl, halo, or CN,
B is halo, hydroxy, CN, C₁₋₄alkyl, or C₁₋₄haloalkyl,
E is halo, CN, NO₂, hydroxy, C₁₋₇alkyl, C₁₋₇alkoxy, C₁₋₇haloalkyl, C₁₋₇hydroxyalkyl, C₁₋₇cyanoalkyl, C₁₋₇haloalkoxy, or C₃₋₇cycloalkyl,
or a pharmaceutically acceptable salt thereof

A certain embodiment of the invention comprises the compound of formula I 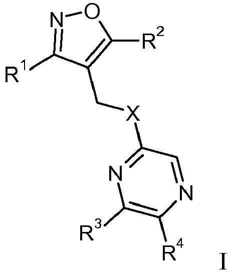 wherein
X is O or NH;
R¹ is phenyl or pyridine-2-yl, wherein the rings may be optionally substituted with halogen;
R² is H or C₁₋₄alkyl;
R³ and R⁴ each are independently
   H,
   -C(O)-R^{a}, wherein R^{a} is hydroxy, C₁₋₇alkoxy or C₁₋₇alkyl,
   -C(O)-NR^{b}R^{c}, wherein R^{b} and R^{c} are each independently hydrogen, C₁₋₇alkyl, optionally substituted with one or more halogen or hydroxy, or are independently from each other -(CH₂)_{z}-C₃₋₇cycloalkyl, optionally substituted by one or more halogen for z being 0 or 1, or are independently from each other heterocyclyl,
   or R³ and R⁴ together form an annelated benzo ring,
or a pharmaceutically acceptable salt thereof.

Preferred compounds of formula I of present invention are
5-(5-methyl-3-phenyl-isoxazol-4-ylmethoxy)-pyrazine-2-carboxylic acid methyl ester,
5-(5-methyl-3-phenyl-isoxazol-4-ylmethoxy)-pyrazine-2-carboxylic acid cyclopropylmethyl-amide,
5-(5-methyl-3-phenyl-isoxazol-4-ylmethoxy)-pyrazine-2-carboxylic acid isopropylamide,
5-(5-methyl-3-phenyl-isoxazol-4-ylmethoxy)-pyrazine-2-carboxylic acid *tert*-butylamide,
5-(5-methyl-3-phenyl-isoxazol-4-ylmethoxy)-pyrazine-2-carboxylic acid cyclopropylamide,
5-(5-methyl-3-phenyl-isoxazol-4-ylmethoxy)-pyrazine-2-carboxylic acid (tetrahydro-pyran-4-yl)-amide,
5-(5-methyl-3-phenyl-isoxazol-4-ylmethoxy)-pyrazine-2-carboxylic acid (4,4-difluoro-cyclohexyl)-amide,
2-(5-methyl-3-phenyl-isoxazol-4-ylmethoxy)-quinoxaline,
5-[(5-methyl-3-phenyl-isoxazol-4-ylmethyl)-amino]-pyrazine-2-carboxylic acid isopropylamide,
5-[(5-methyl-3-phenyl-isoxazol-4-ylmethyl)-amino]-pyrazine-2-carboxylic acid cyclopropylamide,
5-[(5-methyl-3-phenyl-isoxazol-4-ylmethyl)-amino]-pyrazine-2-carboxylic acid (tetrahydropyran-4-yl)-amide,
5-[3-(5-fluoro-pyridin-2-yl)-5-methyl-isoxazol-4-ylmethoxy]-pyrazine-2-carboxylic acid (tetrahydro-pyran-4-yl)-amide,
5-[3-(4-fluoro-phenyl)-isoxazol-4-ylmethoxy]-pyrazine-2-carboxylic acid (2,2,2-trifluoro-ethyl)-amide,
5-[3-(4-fluoro-phenyl)-isoxazol-4-ylmethoxy]-pyrazine-2-carboxylic acid cyclopropylmethyl-amide,
5-[3-(4-fluoro-phenyl)-isoxazol-4-ylmethoxy]-pyrazine-2-carboxylic acid isopropylamide,
5-[3-(4-chloro-phenyl)-isoxazol-4-ylmethoxy]-pyrazine-2-carboxylic acid isopropylamide,
5-[3-(4-chloro-phenyl)-isoxazol-4-ylmethoxy]-pyrazine-2-carboxylic acid ((S)-2-hydroxy-1-methyl-ethyl)-amide,
5-[3-(4-chloro-phenyl)-isoxazol-4-ylmethoxy]-pyrazine-2-carboxylic acid ((R)-2-hydroxy-1-methyl-ethyl)-amide,
5-(3-pyridin-2-yl-isoxazol-4-ylmethoxy)-pyrazine-2-carboxylic acid (tetrahydro-pyran-4-yl)-amide,
5-(3-pyridin-2-yl-isoxazol-4-ylmethoxy)-pyrazine-2-carboxylic acid isopropylamide or 5-(3-pyridin-2-yl-isoxazol-4-ylmethoxy)-pyrazine-2-carboxylic acid cyclopropylamide

The present compounds of formula I (X = O) and their pharmaceutically acceptable salts can be prepared by a process comprising the steps of:
a) reacting a compound of formula II: 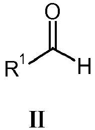 with hydroxylamine hydrochloride in a suitable solvent, such as ethanol and water in the presence of a base, such as aqueous sodium hydroxide to give a compound of formula III: 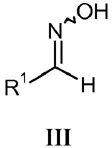
b) reacting the compound of formula III with a chlorinating agent such as N-chlorosuccinimide in a suitable solvent, such as DMF to give a compound of formula IV: 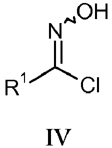
c1) and then either reacting the compound of formula IV with a compound of formula V: 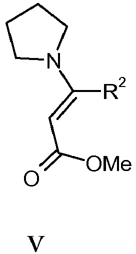 in the presence of a suitable base, such as triethylamine, in a suitable solvent, such as chloroform, or alternatively
c2) reacting the compound of formula IV with a compound of formula VI: 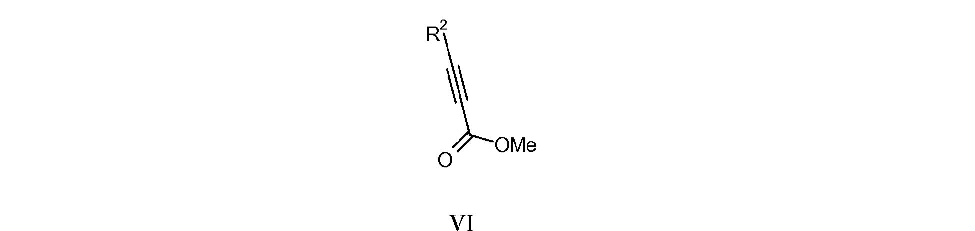 in the presence of a suitable base, such as triethylamine, in a suitable solvent, such as diethylether to give a compound of formula VII: 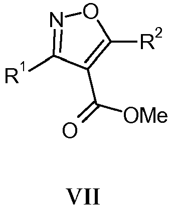
d) reacting a compound of formula VII with a reducing agent, such as lithiumaluminiumhydride, in a suitable solvent, such as THF to give a compound of formula VIII: 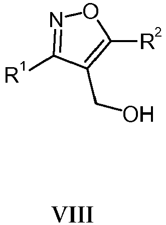
   i) with a compound of formula IX : 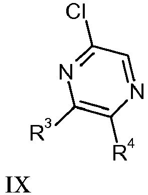 in the presence of a suitable base, such as sodium hydride, in a suitable solvent, such as THF to give a compound of formula I:

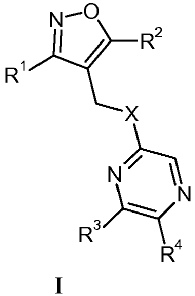

The present compounds of formula I (X = NH) and their pharmaceutically acceptable salts can be prepared by a process comprising the steps of:
j) reacting a compound of formula VIII: 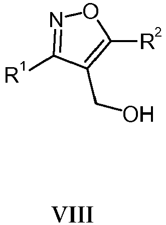 with phthalimide in the presence of triphenylphosphine and diethylazodicarboxylate, in a suitable solvent, such as THF to give a compound of formula X: 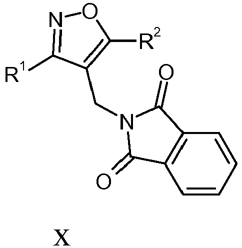
g) reacting the compound of formula X with hydrazine, to give a compound of formula XI: 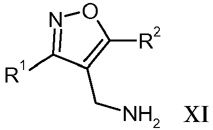 with a compound of formula IX : 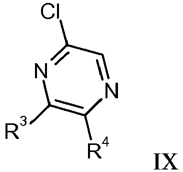 in the presence of a suitable base, such as sodium hydride, in a suitable solvent, such as THF to give a compound of formula I:

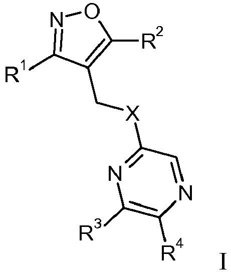

The following scheme describes the processes for preparation of compounds of formula I (X = O and NH) in more detail.

In accordance with Scheme 1, compounds of formula I can be prepared following standards methods. 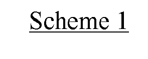 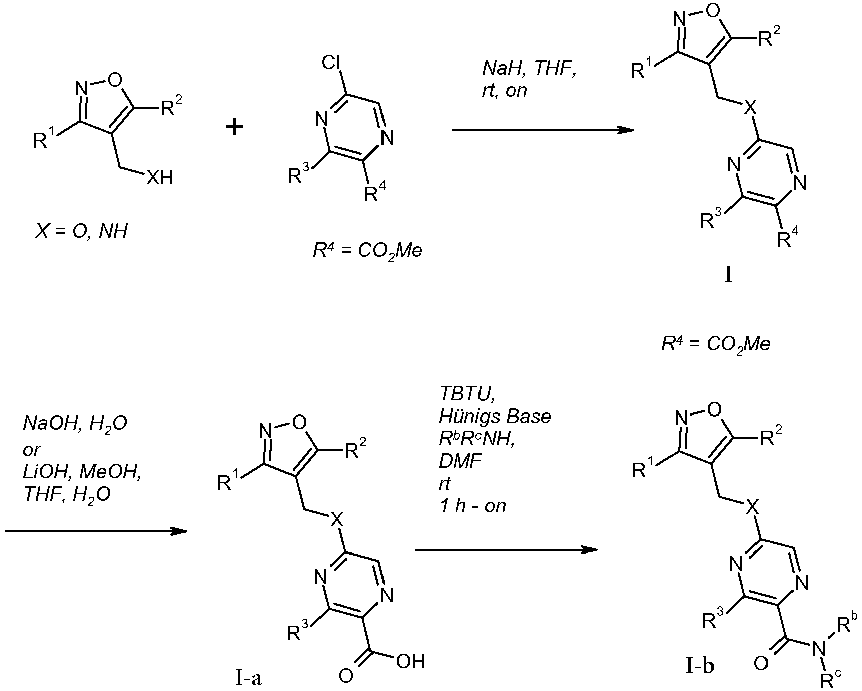
on = overnight
rt = room temperature
DMF = N,N-dimethylformamide
TBTU = O-(Benzotriazol-1-yl)-N,N,N',N'-tetramethyluronium tetrafluoroborate

As mentioned earlier, the compounds of formula I and their pharmaceutically usable salts possess valuable pharmacological properties. It has been found that the compounds of the present invention are ligands for GABA A receptors containing the α5 subunit and are therefore useful in the therapy where cognition enhancement is required.

The compounds were investigated in accordance with the test given hereinafter:

### Membrane preparation and binding assay

The affinity of compounds at GABA A receptor subtypes was measured by competition for [3H]flumazenil (85 Ci/mmol; Roche) binding to HEK293 cells expressing rat (stably transfected) or human (transiently transfected) receptors of composition α1β3γ2, α2β3γ2, α3β3γ2 and α5β3γ2.

Cell pellets were suspended in Krebs-tris buffer (4.8 mM KCl, 1.2 mM CaCl2, 1.2 mM MgCl₂, 120 mM NaCl, 15 mM Tris; pH 7.5; binding assay buffer), homogenized by polytron for ca. 20 sec on ice and centrifuged for 60 min at 4 °C (50000 g; Sorvall, rotor: SM24 = 20000 rpm). The cell pellets were resuspended in Krebs-tris buffer and homogenized by polytron for ca. 15 sec on ice. Protein was measured (Bradford method, Bio-Rad) and aliquots of 1 mL were prepared and stored at -80 °C.

Radioligand binding assays were carried out in a volume of 200 mL (96-well plates) which contained 100 mL of cell memebranes, [3H]flumazenil at a concentration of 1 nM for α1, α2, α3 subunits and 0.5 nM for α5 subunits and the test compound in the range of 10-10⁻³ x 10⁻⁶ M. Nonspecific binding was defined by 10⁻⁵ M diazepam and typically represented less than 5% of the total binding. Assays were incubated to equilibrium for 1 hour at 4 °C and harvested onto GF/C uni-filters (Packard) by filtration using a Packard harvester and washing with ice-cold wash buffer (50 mM Tris; pH 7.5). After drying, filter-retained radioactivity was detected by liquid scintillation counting. Ki values were calculated using Excel-Fit (Microsoft) and are the means of two determinations.

The compounds of the accompanying examples were tested in the above described assay, and the preferred compounds were found to possess a Ki value for displacement of [3H]flumazenil from α5 subunits of the rat GABA A receptor of 100 nM or less. Most preferred are compounds with a Ki (nM) < 35. In a preferred embodiment the compounds of the invention are binding selective for the α5 subunit relative to the α1, α2 and α3 subunit.

Representative test results are shown in the table below:

**Table 1:**

| Example | hKi (nM) | Example | hKi (nM) | Example | hKi (nM) | Example | hKi (nM) |
|---|---|---|---|---|---|---|---|
| 1 | 29 | 7 | 10.6 | 13 | 24 | 19 | 14.3 |
| 2 | 3.1 | 8 | 7.4 | 14 | 21 | 20 | 9.6 |
| 3 | 1.2 | 9 | 9.6 | 15 | 11 | 21 | 13.7 |
| 4 | 10.8 | 10 | 10.4 | 16 | 11.6 | | |
| 5 | 1.9 | 11 | 12.1 | 17 | 32 | | |
| 6 | 1.2 | 12 | 0.3 | 18 | 7.4 | | |

| | | | | | | | |
|---|---|---|---|---|---|---|---|
| "h" in "hKi" means "human". | | | | | | | |

The compounds of formula I as well as their pharmaceutically usable acid addition salts can be used as medicaments, e.g. in the form of pharmaceutical preparations. The pharmaceutical preparations can be administered orally, e.g. in the form of tablets, coated tablets, dragees, hard and soft gelatine capsules, solutions, emulsions or suspensions. The administration can, however, also be effected rectally, e.g. in the form of suppositories, or parenterally, e.g. in the form of injection solutions.

The compounds of formula I and their pharmaceutically usable acid addition salts can be processed with pharmaceutically inert, inorganic or organic excipients for the production of tablets, coated tablets, dragees and hard gelatine capsules. Lactose, corn starch or derivatives thereof, talc, stearic acid or its salts etc can be used as such excipients e.g. for tablets, dragees and hard gelatine capsules. Suitable excipients for soft gelatine capsules are e.g. vegetable oils, waxes, fats, semisolid and liquid polyols etc.

Suitable excipients for the manufacture of solutions and syrups are e.g. water, polyols, saccharose, invert sugar, glucose etc.

Suitable excipients for injection solutions are e.g. water, alcohols, polyols, glycerol, vegetable oils etc.

Suitable excipients for suppositories are e.g. natural or hardened oils, waxes, fats, semiliquid or liquid polyols etc.

Moreover, the pharmaceutical preparations can contain preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

The dosage can vary within wide limits and will, of course, be fitted to the individual requirements in each particular case. In general, in the case of oral administration a daily dosage of about 0.1 to 1000 mg per person of a compound of general formula I should be appropriate, although the above upper limit can also be exceeded when necessary.

The following examples illustrate the present invention without limiting it. All temperatures are given in degrees Celsius.

### Example A

Tablets of the following composition are manufactured in the usual manner:

| | mg/tablet |
|---|---|
| Active substance | 5 |
| Lactose | 45 |
| Corn starch | 15 |
| Microcrystalline cellulose | 34 |
| Magnesium stearate | 1 |
| Tablet weight | 100 |

### Example B

Capsules of the following composition are manufactured:

| | mg/capsule |
|---|---|
| Active substance | 10 |
| Lactose | 155 |
| Corn starch | 30 |
| Talc | 5 |
| Capsule fill weight | 200 |

The active substance, lactose and corn starch are firstly mixed in a mixer and then in a comminuting machine. The mixture is returned to the mixer, the talc is added thereto and mixed thoroughly. The mixture is filled by machine into hard gelatine capsules.

### Example C

Suppositories of the following composition are manufactured:

| | mg/supp. |
|---|---|
| Active substance | 15 |
| Suppository mass | 1285 |
| Total | 1300 |

The suppository mass is melted in a glass or steel vessel, mixed thoroughly and cooled to 45 °C. Thereupon, the finely powdered active substance is added thereto and stirred until it has dispersed completely. The mixture is poured into suppository moulds of suitable size, left to cool, the suppositories are then removed from the moulds and packed individually in wax paper or metal foil.

The following examples 1 - 21 are provided for illustration of the invention. They should not be considered as limiting the scope of the invention, but merely as being representative thereof.

### Example 1

### 5-(5-Methyl-3-phenyl-isoxazol-4-ylmethoxy)-pyrazine-2-carboxylic acid methyl ester

To a solution of (5-methyl-3-phenyl-isoxazol-4-yl)-methanol (1.24 g, 6.55 mmol) in THF (12 mL) was added sodium hydride (55% dispersion in mineral oil, 0.31 g, 7.2 mmol) at 0 °C. The reaction mixture was stirred for 30 min while it was allowed to warm up to room temperature. Methyl 5-chloropyrazine-2-carboxylate (1.36 g, 7.86 mmol) was added and stirring was continued for 2 h. Water (10 mL) was added and the mixture was extracted with ethyl acetate (40 mL). The combined organic layers were washed with brine (10 mL) and dried over sodium sulfate. Concentration and purification of the residue by chromatography afforded the title compound (SiO₂, heptane:ethyl acetate = 80:20 to 50:50, 1.00 g, 47%) which was obtained as a light yellow oil. MS: m/e = 326.2 [M+H]⁺.

### Example 2

### 5-(5-Methyl-3-phenyl-isoxazol-4-ylmethoxy)-pyrazine-2-carboxylic acid cyclopropylmethyl-amide

### a) 5-(5-Methyl-3-phenyl-isoxazol-4-ylmethoxy)-pyrazine-2-carboxylic acid

To a solution of 5-(5-methyl-3-phenyl-isoxazol-4-ylmethoxy)-pyrazine-2-carboxylic acid methyl ester (1.00 g, 3.07 mmol) (1.0 g, 1.69 mmol) in ethanol (10 mL) was added aqueous sodium hydroxide (1 N, 6.2 mL). After heating at 60 °C for 30 min it was cooled to ambient temperature and aqueous sodium carbonate (2 M, 50 mL) added. Addition of aqueous sodium hydroxide (1 M, 50 mL) was followed by extraction with tert-butylmethylether. The aqueous phase was acidified with aqueous hydrogen chloride (25%) to pH=2 and extracted with tert-butylmethylether and ethyl acetate. The combined organic layers were dried over sodium sulfate and concentration afforded the title compound (450 mg, 86%) as a white foam. MS: m/e = 310.5 [M-H]⁻.

### b) 5-(5-Methyl-3-phenyl-isoxazol-4-ylmethoxy)-pyrazine-2-carboxylic acid cyclopropylmethyl-amide

To a solution of 5-(5-methyl-3-phenyl-isoxazol-4-ylmethoxy)-pyrazine-2-carboxylic acid (150 mg, 0.48 mmol) in DMF (2 mL) were added 2-(1H-benzotriazole-1-yl)-1,1,3,3-tetramethyluronium tetrafluoroborate (170 mg, 0.55 mmol), N,N-diisopropyl ethyl amine (410 L, 2.4 mmol) and aminomethylcyclopropane (41 mg, 0.58 mmol). The resulting reaction mixture was stirred for 30 min at room temperature and diluted with water. The mixture was then extracted with ethyl acetate and the combined organic layers washed with aqueous sodium carbonate (saturated) and dried over sodium sulfate. Concentration and purification by chromatography (SiO₂, heptane:ethyl acetate = 90:10 to 60:40) afforded the title compound (117 mg, 67%) which was obtained as an off-white solid. MS: m/e = 365.3 [M+H]⁺.

### Example 3

### 5-(5-Methyl-3-phenyl-isoxazol-4-ylmethoxy)-pyrazine-2-carboxylic acid isopropylamide

As described for example 2b, 5-(5-methyl-3-phenyl-isoxazol-4-ylmethoxy)-pyrazine-2-carboxylic acid (100 mg, 0.32 mmol), was converted, using isopropylamine instead of aminomethylcyclopropane, to the title compound (SiO₂, heptane:ethyl acetate = 90:10 to 60:40, 37 mg, 33%) which was obtained as an off-white solid. MS: m/e = 353.2 [M+H]⁺.

### Example 4

### 5-(5-Methyl-3-phenyl-isoxazol-4-ylmethoxy)-pyrazine-2-carboxylic acid tert-butylamide

As described for example 2b, 5-(5-methyl-3-phenyl-isoxazol-4-ylmethoxy)-pyrazine-2-carboxylic acid (100 mg, 0.32 mmol) was converted using tert-butylamine instead of aminomethylcyclopropane, to the title compound (SiO₂, heptane:ethyl acetate = 90:10 to 60:40, 24 mg, 20%) which was obtained as a colorless gum. MS: m/e = 367.2 [M+H]⁺.

### Example 5

### 5-(5-Methyl-3-phenyl-isoxazol-4-ylmethoxy)-pyrazine-2-carboxylic acid cyclopropylamide

As described for example 2b, 5-(5-methyl-3-phenyl-isoxazol-4-ylmethoxy)-pyrazine-2-carboxylic acid (100 mg, 0.32 mmol) was converted, using cyclopropylamine instead of aminomethylcyclopropane, to the title compound (SiO₂, heptane:ethyl acetate = 90:10 to 60:40, 32 mg, 28%) which was obtained as a white solid. MS: m/e = 351.3 [M+H]⁺.

### Example 6

### 5-(5-Methyl-3-phenyl-isoxazol-4-ylmethoxy)-pyrazine-2-carboxylic acid (tetrahydro-pyran-4-yl)-amide

As described for example 2b, 5-(5-methyl-3-phenyl-isoxazol-4-ylmethoxy)-pyrazine-2-carboxylic acid (125 mg, 0.40 mmol) was converted, using 4-aminotetrahydropyran instead of aminomethylcyclopropane to the title compound (SiO₂, heptane:ethyl acetate = 70:30 to 40:60, 73 mg, 46%) which was obtained as a white solid. MS: m/e = 395.1 [M+H]⁺.

### Example 7

### 5-(5-Methyl-3-phenyl-isoxazol-4-ylmethoxy)-pyrazine-2-carboxylic acid (4,4-difluoro-cyclohexyl)-amide

As described for example 2b, 5-(5-methyl-3-phenyl-isoxazol-4-ylmethoxy)-pyrazine-2-carboxylic acid (100 mg, 0.32 mmol) was converted, using 4,4-difluorocyclohexylamine instead of aminomethylcyclopropane to the title compound (SiO₂, heptane:ethyl acetate = 90:10 to 60:40, 37 mg, 27%) which was obtained as a white solid. MS: m/e = 429.2 [M+H]⁺.

### Example 8

### 2-(5-Methyl-3-phenyl-isoxazol-4-ylmethoxy)-quinoxaline

To a solution of (5-methyl-3-phenyl-isoxazol-4-yl)-methanol (100 mg, 0.53 mmol) in THF (6 mL) was added 2- hydroxyquinoxaline (77 mg, 0.53 mmol) and tributyl phosphine (206 L, 0.79 mmol) at ambient temperature under an argon atmosphere. After cooling to 0 °C, N,N,N',N'-tetramethylazodicarboxamide (137 mg, 0.79 mmol) was added. The resulting orange solution was stirred for 16 h at ambient temperature followed by 2.5 h at 50 °C. Then triphenylphosphine (208 mg, 0.79 mmol), 2- hydroxyquinoxaline (77 mg, 0.53 mmol) and diethyl azodicarboxylate (127 L, 0.79 mmol) were added and the reaction mixture was stirred for 4 h at 50 °C. The reaction mixture was then evaporated. Purification by chromatography (SiO₂, heptane:ethyl acetate = 95:5 to 0:100) afforded the title compound (67 mg, 40%) as a white solid. MS: m/e = 318.2 [M+H]⁺.

### Example 9

### 5-[(5-Methyl-3-phenyl-isoxazol-4-ylmethyl)-amino]-pyrazine-2-carboxylic acid isopropylamide

### a) 5-[(5-Methyl-3-phenyl-isoxazol-4-ylmethyl)-amino]-pyrazine-2-carboxylic acid methyl ester

A solution of (5-methyl-3-phenyl-4-isoxazolyl)methylamine (2.4 g, 12.7 mmol) and 5-chloropyrazine-2-carboxylic acid methyl ester (2.2 g, 12.7 mmol) in DMSO (15 mL) was heated with microwave irradiation to 160 °C for 30 min. After cooling to room temperature the reaction mixture was extracted with ethyl acetate and the combined extracts washed with water. The organic phase was dried over sodium sulfate, concentrated and chromatographed (SiO₂, heptane:ethyl acetate = 100:0 to 20:80). The oily product obtained was triturated with diisopropylether and ethyl acetate to afford the title compound (3.5 g, 84%) as an off-white solid. MS: m/e = 325.4[M+H]⁺.

### b) 5-[(5-Methyl-3-phenyl-isoxazol-4-ylmethyl)-amino]-pyrazine-2-carboxylic acid isopropylamide

To a solution of isopropylamine (0.69 mL, 8 mmol) in dioxane (5 mL) was added dropwise trimethylaluminum solution (2M solution in hexane, 4 mL, 8 mmol). After stirring for 1 h at room temperature a suspension of 5-[(5-methyl-3-phenyl-isoxazol-4-ylmethyl)-amino]-pyrazine-2-carboxylic acid methyl ester (650 mg, 2 mmol) in dioxane (5 mL) was added. The reaction mixture was stirred at 90 °C for 90 min, cooled to room temperature and poured into water. Extraction with ethyl acetate and washing with saturated aqueous Seignette salt solution followed by drying of the organic phase over sodium sulfate and evaporation afforded an oil. Purification by chromatography (SiO₂, heptane:ethyl acetate = 100:0 to 20:80) afforded the title compound (600 mg, 85%) as a white solid. MS: m/e = 352.3 [M+H]⁺.

### Example 10

### 5-[(5-Methyl-3-phenyl-isoxazol-4-ylmethyl)-amino]-pyrazine-2-carboxylic acid cyclopropylamide

As described for example 9b, 5-[(5-methyl-3-phenyl-isoxazol-4-ylmethyl)-amino]-pyrazine-2-carboxylic acid methyl ester (650 mg, 2 mmol) was converted, using cyclopropylamine instead of isopropylamine, to the title compound (600 mg, 86%) which was obtained as a white solid. MS: m/e = 394.3 [M+H]⁺.

### Example 11

### 5-[(5-Methyl-3-phenyl-isoxazol-4-ylmethyl)-amino]-pyrazine-2-carboxylic acid (tetrahydropyran-4-yl)-amide

As described for example 9b, 5-[(5-methyl-3-phenyl-isoxazol-4-ylmethyl)-amino]-pyrazine-2-carboxylic acid methyl ester (650 mg, 2 mmol) was converted, using 4-aminotetrahydropyran instead of isopropylamine, to the title compound (640 mg, 81%) which was obtained as a white solid. MS: m/e = 350.4 [M+H]⁺.

### Example 12

### 5-[3-(5-Fluoro-pyridin-2-yl)-5-methyl-isoxazol-4-ylmethoxy]-pyrazine-2-carboxylic acid (tetrahydro-pyran-4-yl)-amide

### a) 5-Fluoro-pyridine-2-carbaldehyde oxime

To a solution of 5-fluoro-2-formylpyridine (5.0 g, 41 mmol) and hydroxylamine hydrochloride (3.06 g, 44 mmol) in ethanol (3.2 mL) and water (9.6 mL) was added ice (18.6 g). Then a solution of NaOH (4.0 g, 100 mmol) in water (4.6 mL) was added dropwise over 10 min keeping the temperature between -5 °C and 5 °C. The reaction mixture was then stirred at room temperature for 30 min. Then HCl (4 N) was added to acidify the mixture and the resulting precipitate was filtered off and washed with water to afford the title compound (4.41 g, 79%) as a light brown solid. MS: m/e = 141.0 [M+H]⁺.

### b) 3-(5-Fluoro-pyridin-2-yl)-5-methyl-isoxazole-4-carboxylic acid ethyl ester

To a suspension of N-chlorosuccinimide (4.63 g, 35 mmol) in chloroform (21 mL) was added pyridine (0.28 mL, 3.5 mmol) and a solution of 5-fluoro-pyridine-2-carbaldehyde oxime (4.86 g, 35 mmol) in chloroform (110 mL) during 15 min at room temperature. After stirring for 30 min at this temperature a solution of ethyl (E)-3-(1-pyrrolidino)-2-butenoate (6.36 g, 35 mmol) in chloroform (4.4 mL) was added. The resulting suspension was warmed to 50 °C and a solution of triethylamine (4.83 mL, 35 mmol) in chloroform (4.4 mL) was added dropwise over a period of 30 min. Stirring was continued for 1.5 h at 50 °C and then cooled to ambient temperature. The solution was then diluted with ice-water (200 mL) and the aqueous layers were extracted with dichloromethane (50 mL) and dried over sodium sulfate and evaporation to give a dark brown oil. Purification by chromatography (SiO₂, heptane:ethyl acetate = 100:0 to 20:80) afforded the title compound (5.83 g, 67%) as yellow oil. MS: m/e = 251.1 [M+H]⁺.

### c) [3-(5-Fluoro-pyridin-2-yl)-5-methyl-isoxazol-4-yl]-methanol 1

To a solution of 3-(5-fluoro-pyridin-2-yl)-5-methyl-isoxazole-4-carboxylic acid ethyl ester (2.5 g, 10 mmol) in dry THF (34 mL), cooled to 0 °C, was added lithiumaluminumhydride (209 mg, 2.3 mmol) portionwise. After allowing to warm up to room temperature over 1 h, the mixture was cooled to 0 °C and water (0.2 mL) was added carefully followed by aqueous sodium hydroxide (15%, 0.2 mL) and water (0.6 mL). The resulting suspension was stirred for 4 h at ambient temperature and filtered over Hyflo®. The filtrate was then concentrated and purification by chromatography (SiO₂, heptane:ethyl acetate = 50:50 to 0:100) afforded the title compound (1.47 g, 71%) as a light yellow solid. MS: m/e = 209.1 [M+H]⁺.

### d) 5-[3-(5-Fluoro-pyridin-2-yl)-5-methyl-isoxazol-4-ylmethoxy]-pyrazine-2-carboxylic acid

As decribed for example 1, [3-(5-fluoro-pyridin-2-yl)-5-methyl-isoxazol-4-yl]-methanol (1.0 g, 4.8 mmol), instead of (5-methyl-3-phenyl-isoxazol-4-yl)-methanol, was converted to 5-[3-(5-fluoro-pyridin-2-yl)-5-methyl-isoxazol-4-ylmethoxy]-pyrazine-2-carboxylic acid methyl ester (667 mg) which was obtained as an ∼1:1 mixture with the starting alcohol after purification by chromatography (SiO₂, heptane:ethyl acetate = 50:50 to 0:100) as a light yellow solid. MS: m/e = 345.2 [M+H]⁺. To a solution of the product mixture (655 mg, 0.86 mmol) in THF (2.2 mL), water (2.2 mL) and methanol (0.4 mL) was added lithium hydroxide monohydrate (72 mg, 1.7 mmol) and the resulting mixture stirred at room temperature for 72 h. The mixture was then evaporated and aqueous sodium hydroxide (IN) added and a white precipitate formed which was filtered off (25 mg) and the filtrate was extracted with ethyl acetate. The aqueous phase was then acidified with HCl (4 N) and the precipitate was filtered off (39 mg). The combined precipitates were combined to afford the title compound (64 mg, 23%) which was obtained as an off-white solid. MS: m/e = 329.2 [M-H]⁻.

### e) 5-[3-(5-Fluoro-pyridin-2-yl)-5-methyl-isoxazol-4-ylmethoxy]-pyrazine-2-carboxylic acid (tetrahydro-pyran-4- yl)-amide

As described for example 2b, 5-[3-(5-fluoro-pyridin-2-yl)-5-methyl-isoxazol-4-ylmethoxy]-pyrazine-2-carboxylic acid (44 mg, 13.3 mmol), instead of 5-(5-methyl-3-phenyl-isoxazol-4-ylmethoxy)-pyrazine-2-carboxylic acid, was converted, using 4-aminotetrahydropyran instead of aminomethylcyclopropane, to the title compound (SiO₂, ethyl acetate then DCM:MeOH = 90:10, 31 mg, 56%) which was obtained as a white solid. MS: m/e = 414.2 [M+H]⁺.

### Example 13

### 5-[3-(4-Fluoro-phenyl)-isoxazol-4-ylmethoxy]-pyrazine-2-carboxylic acid (2,2,2-trifluoro-ethyl)-amide

### a) (E)- and/or (Z)-4-Fluoro-benzaldehyde oxime

As described for example 12a, 4-fluorobenzaldehyde (24.8 g, 200 mmol) was converted, instead of 5-fluoro-2-formylpyridine, to the title compound (23.3 g, 84%) which was obtained as a white solid. MS: m/e = 139.1 [M]⁺.

### b) (E)- and/or (Z)-N-Hydroxy-4-fluoro-benzenecarboximidoyl chloride

To a solution of (E)- and/or (Z)-4-fluoro-benzaldehyde oxime (100 g, 719 mmol) in DMF (500 mL) was added N-chlorosuccinimide (110 g, 791 mmol) portionwise keeping the temperature below 70 °C. The reaction mixture was stirred at room temperature for 2.5 h and then extracted with tert-butyl methyl ether to afford the title compound (125 g, 100%) which was obtained as a yellow oil. MS: m/e = 173.1 [M]⁺.

### c) 3-(4-Fluoro-phenyl)-isoxazole-4-carboxylic acid ethyl ester

To a solution of (E)- and/or (Z)-N-hydroxy-4-fluoro-benzenecarboximidoyl chloride (50 g, 241 mmol) in diethylether (1 L) was added a solution of ethyl 3-(N,N- dimethylamino)acrylate (87 mL, 601 mmol) and triethylamine (49 mL, 349 mmol) in diethylether (1 L). The resulting mixture was then stirred for 14 h at room temperature and evaporated. Purification by chromatography (SiO₂, heptane:ethyl acetate = 100:0 to 4:1) afforded the title product (50.2 g, 88%) which was obtained as a light yellow solid. MS: m/e = 236.1 [M+H]⁺.

### d) 3-(4-Fluoro-phenyl)-isoxazole-4-carboxylic acid

To a solution of 3-(4-fluoro-phenyl)-isoxazole-4-carboxylic acid ethyl ester (849 g, 208 mmol) in ethanol (215 mL) was added aqueous sodium hydroxide (2 N, 161 mL, 323 mmol) and the resulting mixture stirred overnight at room temperature. The mixture was then acidified with HCl solution (4 N, 85 mL) to pH 2-3. The precipitate was then filtered off and dissolved in THF (700 mL) and then washed with saturated sodium chloride solution. The aqueous phase was then extracted with ethyl acetate and THF (1:1, 300 mL) and the combined organic phases dried over sodium sulfate and evaporated to afford the title compound (40.8 g, 94%) which was obtained as an orange solid. MS: m/e = 206.1 [M-H]⁻.

### e) [3-(4-Fluoro-phenyl)-isoxazol-4-yl]-methanol

To a solution of 3-(4-fluoro-phenyl)-isoxazole-4-carboxylic acid (40 g, 193 mmol) in THF (400 mL) at - 10 °C was added triethylamine (27.1 mL, 193 mmol) and then a solution of ethylchloroformate (18.8 mL, 193 mmol) in THF (120 mL) added keeping the temperature below - 5 °C. After 1 h the mixture was filtered and the filtrate cooled to - 10 °C and a suspension of sodiumborohydride (19 g, 483 mmol) in water (120 mL) added over 15 minutes keeping the temperature below - 5 °C. The mixture was then allowed to warm up to room temperature over 2 h and diluted with aqueous sodium hydroxide (1 N, 700 mL) and extracted with tert-butylmethylether. The combined organic layers were then washed with water and brine, dried over sodium sulfate and evaporated. Purification by chromatography (SiO₂, heptane:ethyl acetate =1:1) afforded the title product (20.1 g, 54%) which was obtained as white solid. MS: m/e = 194.1 [M+H]⁺.

### f) 5-[3-(4-Fluoro-phenyl)-isoxazol-4-ylmethoxy]-pyrazine-2-carboxylic acid methyl ester

As decribed for example 1, [3-(4-fluoro-phenyl)-isoxazol-4-yl]-methanol (150 mg, 0.78 mmol), instead of (5-methyl-3-phenyl-isoxazol-4-yl)-methanol, was converted to the title compound (153 mg, 60%) which was obtained as a white solid. MS: m/e = 388.1 [M+OAc]⁺.

### g) 5-[3-(4-Fluoro-phenyl)-isoxazol-4-ylmethoxy]-pyrazine-2-carboxylic acid (2,2,2-trifluoro-ethyl)-amide

As described for example 9b, 5-[3-(4-fluoro-phenyl)-isoxazol-4-ylmethoxy]-pyrazine-2-carboxylic acid methyl ester (75 mg, 0.23 mmol), instead of 5-[(5-methyl-3-phenyl-isoxazol-4-ylmethyl)-amino]-pyrazine-2-carboxylic acid methyl ester, was converted, using 2,2,2-trifluoroethylamine instead of isopropylamine, to the title compound (64 mg, 71 %) which was obtained as a light yellow solid. MS: m/e = 397.2 [M+H]⁺.

### Example 14

### 5-[3-(4-Fluoro-phenyl)-isoxazol-4-ylmethoxy]-pyrazine-2-carboxylic acid cyclopropylmethyl-amide

As described for example 13g, 5-[3-(4-fluoro-phenyl)-isoxazol-4-ylmethoxy]-pyrazine-2-carboxylic acid methyl ester (75 mg, 0.23 mmol) was converted, using aminomethylcyclopropane instead of 2,2,2-trifluoroethylamine, to the title compound (44 mg, 52%) which was obtained as a light yellow solid. MS: m/e = 427.0 [M+OAc]⁺.

### Example 15

### 5-[3-(4-Fluoro-phenyl)-isoxazol-4-ylmethoxy]-pyrazine-2-carboxylic acid isopropylamide

As described for example 13g, 5-[3-(4-fluoro-phenyl)-isoxazol-4-ylmethoxy]-pyrazine-2-carboxylic acid methyl ester (100 mg, 0.3 mmol) was converted, using isopropylamine instead of 2,2,2-trifluoroethylamine, to the title compound (81 mg, 75%) which was obtained as a light yellow solid. MS: m/e = 415.1 [M+OAc]⁺.

### Example 16

### 5-[3-(4-Chloro-phenyl)-isoxazol-4-ylmethoxy]-pyrazine-2-carboxylic acid isopropylamide

### a) (E)- and/or (Z)-4-Chloro-benzaldehyde oxime

As described for example 13a, 4-chlorobenzaldehyde (25.0 g, 178 mmol) was converted, instead of 4-fluorobenzaldehyde, to the title compound (27.0 g, 97%) which was obtained as an off-white solid. MS: m/e = 155.1 [M]⁺.

### b) (E)- and/or (Z)-N-Hydroxy-4-chloro-benzenecarboximidoyl chloride

As described for example 13b, (E)- and/or (Z)-4-chloro-benzaldehyde oxime (27.0 g, 173 mmol) was converted, instead of (E)- and/or (Z)-4-fluoro-benzaldehyde oxime, to the title compound (28.4 g, 86%) which was obtained as a light yellow solid. MS: m/e = 189.1 [M]⁺.

### c) 3-(4-Chloro-phenyl)-isoxazole-4-carboxylic acid ethyl ester

As described for example 13c, (E)- and/or (Z)-N-hydroxy-4-chloro-benzenecarboximidoyl chloride (58.0 g, 250.3 mmol) was converted, instead of (E)- and/or (Z)-N-hydroxy-4-fluoro-benzenecarboximidoyl chloride, to the title compound (57 g, 91%) which was obtained as a white solid. MS: m/e = 252.1 [M+H]⁺.

### d) 3-(4-Chloro-phenyl)-isoxazole-4-carboxylic acid

As described for example 13d, 3-(4-chloro-phenyl)-isoxazole-4-carboxylic acid ethyl ester (57.0 g, 226.5 mmol) was converted, instead of 3-(4-fluoro-phenyl)-isoxazole-4-carboxylic acid ethyl ester, to the title compound (50.7 g, 92%) which was obtained as a light yellow solid. MS: m/e = 222.3 [M-H]⁻.

### e) [3-(4-Chloro-phenyl)-isoxazol-4-yl]-methanol

As described for example 13e, 3-(4-chloro-phenyl)-isoxazole-4-carboxylic acid (40.0 g, 178.9 mmol) was converted, instead of 3-(4-fluoro-phenyl)-isoxazole-4-carboxylic acid, to the title compound (17.3 g, 46%) which was obtained as a light green solid. MS: m/e = 210.1 [M+H]⁺.

### f) 5-[3-(4-Chloro-phenyl)-isoxazol-4-ylmethoxy]-pyrazine-2-carboxylic acid methyl ester

As decribed for example 1, [3-(4-chloro-phenyl)-isoxazol-4-yl]-methanol (1.5 g, 7.2 mmol), instead of [3-(4-fluoro-phenyl)-isoxazol-4-yl]-methanol, was converted to the title compound (1.7 g, 68%) which was obtained as a white solid. MS: m/e = 404.1 [M+OAc]⁺.

### g) 5-[3-(4-Chloro-phenyl)-isoxazol-4-ylmethoxy]-pyrazine-2-carboxylic acid isopropylamide

As described for example 15, 5-[3-(4-chloro-phenyl)-isoxazol-4-ylmethoxy]-pyrazine-2-carboxylic acid methyl ester (100 mg, 0.29 mmol), instead of 5-[3-(4-fluoro-phenyl)-isoxazol-4-ylmethoxy]-pyrazine-2-carboxylic acid methyl ester, was converted to the title compound (1.3 mg, 1%) which was obtained as a light yellow solid. MS: m/e = 431.1 [M+OAc]⁺.

### Example 17

### 5-[3-(4-Chloro-phenyl)-isoxazol-4-ylmethoxy]-pyrazine-2-carboxylic acid ((S)-2-hydroxy-1-methyl-ethyl)-amide

### a) 5-[3-(4-Chloro-phenyl)-isoxazol-4-ylmethoxy]-pyrazine-2-carboxylic acid

To a solution of 5-[3-(4-chloro-phenyl)-isoxazol-4-ylmethoxy]-pyrazine-2-carboxylic acid methyl ester (1.0 g, 2.89 mmol) in THF (6.3 mL), water (6.3 mL) and methanol (1.75 mL) was added lithium hydroxide monohydrate (141.4 mg, 5.78 mmol) and the resulting mixture stirred at room temperature for 2 h and then acidified with HCl (4 N) and extracted with ethyl acetate. The combined organic layers were then washed with water and brine, dried over sodium sulfate and evaporated to afford the title product (360 mg, 38%) which was obtained as white solid. MS: m/e = 330.0 [M-H]⁻.

### b) (5-[3-(4-Chloro-phenyl)-isoxazol-4-ylmethoxy]-pyrazine-2-carboxylic acid ((S)-2-hydroxy-1-methyl-ethyl)-amide

To a solution of 5-[3-(4-chloro-phenyl)-isoxazol-4-ylmethoxy]-pyrazine-2-carboxylic acid (80 mg, 0.24 mmol) and D-alaninol (22.8 L, 0.29 mmol) in THF (7 mL) was added 1-hydroxybenzotriazole hydrate (37.3 mg, 0.24 mmol), *N*-ethyldiisopropylamine (105.2 L, 0.60 mmol) and *N*-(3-dimethylaminopropyl)-*N'*-ethylcarbodiimide hydrochloride (47.1 mg, 0.24 mmol) at room temperature under nitrogen. The reaction mixture was stirred at room temperature for 72 h. Then the reaction mixture was diluted with water (10 mL), and extracted with ethyl acetate. The combined organic layers were then washed with water and brine, dried over sodium sulfate and evaporated. Purification by chromatography (SiO₂, heptane:ethyl acetate = 1:1 to 2:3) afforded the title product (39 mg, 42%) which was obtained as white solid. MS: m/e = 389.0 [M+H]⁺.

### Example 18

### 5-[3-(4-Chloro-phenyl)-isoxazol-4-ylmethoxy]-pyrazine-2-carboxylic acid ((R)-2-hydroxy-1-methyl-ethyl)-amide

As described for example 17b, 5-[3-(4-chloro-phenyl)-isoxazol-4-ylmethoxy]-pyrazine-2-carboxylic acid (80 mg, 0.24 mmol) was converted, using L-alaninol instead of D-alaninol, to the title compound (44 mg, 47%) which was obtained as a white solid. MS: m/e = 389.0 [M+H]⁺.

### Example 19

### 5-(3-Pyridin-2-yl-isoxazol-4-ylmethoxy)-pyrazine-2-carboxylic acid (tetrahydro-pyran-4-yl)-amide

### a) 3-Pyridin-2-yl-isoxazole-4-carboxylic acid ethyl ester

To a solution of N-chlorosuccinimide (54.7 g, 409 mmol) in DMF (1 L) was added pyridine-2-carbaldoxime (50 g, 409 mmol) portionwise and the resulting mixture was then stirred for 64 h at room temperature. To this solution was then added ethyl 3-(N,N- dimethylamino)acrylate (58.6 g, 409 mmol) and triethylamine (82.9 mL, 819 mmol) in chloroform (10 mL) and the resulting mixture was then stirred for 14 h at room temperature and poured onto a mixture of ice water and HCl (4 N, 100 mL) and extracted with ethylacetate. The organic extract was then washed with water, saturated aqueous sodium hydrogen carbonate solution, brine, dried with sodium sulfate, filtered and evaporated. Purification by distillation afforded the title product (58.9 g, 66%) which was obtained as a light brown liquid. Bp 125-127 °C at 0.4 mbar. MS: m/e = 219.2 [M+H]⁺.

### b) 3-Pyridin-2-yl-isoxazole-4-carboxylic acid

As described for example 17a, 3-pyridin-2-yl-isoxazole-4-carboxylic acid ethyl ester (9.6 g, 44 mmol), instead of 5-[3-(4-chloro-phenyl)-isoxazol-4-ylmethoxy]-pyrazine-2-carboxylic acid methyl ester, was converted to the title compound (6.5 g, 79%) which was obtained as an off-white solid. MS: m/e = 189.3 [M-H]⁻.

### c) (3-Pyridin-2-yl-isoxazol-4-yl)-methanol

As described for example 13e, 3-pyridin-2-yl-isoxazole-4-carboxylic acid (39.0 g, 200 mmol) was converted, instead of 3-(4-fluoro-phenyl)-isoxazole-4-carboxylic acid, to the title compound (26.8 g, 76%) which was obtained as a white solid. MS: m/e = 177.2 [M]⁺.

### d) 5-(3-Pyridin-2-yl-isoxazol-4-ylmethoxy)-pyrazine-2-carboxylic acid methyl ester

As decribed for example 1, (3-pyridin-2-yl-isoxazol-4-yl)-methanol (1.0 g, 5.7 mmol), instead of (5-methyl-3-phenyl-isoxazol-4-yl)-methanol, was converted to the title compound (584 mg, 32%) which was obtained as a white solid. MS: m/e = 313.1 [M+H]⁺.

### e) 5-(3-Pyridin-2-yl-isoxazol-4-ylmethoxy)-pyrazine-2-carboxylic acid

As described for example 17a, 5-(3-pyridin-2-yl-isoxazol-4-ylmethoxy)-pyrazine-2-carboxylic acid methyl ester (432 mg, 1.4 mmol), instead of 5-[3-(4-chloro-phenyl)-isoxazol-4-ylmethoxy]-pyrazine-2-carboxylic acid methyl ester, was converted to the title compound (285 mg, 71 %) which was obtained as a white solid. MS: m/e = 297.1 [M-H]⁻.

### f) 5-(3-Pyridin-2-yl-isoxazol-4-ylmethoxy)-pyrazine-2-carboxylic acid (tetrahydro-pyran-4-yl)-amide

As described for example 2b, 5-(3-pyridin-2-yl-isoxazol-4-ylmethoxy)-pyrazine-2-carboxylic acid (75.4 mg, 2.5 mmol), instead of 5-(5-methyl-3-phenyl-isoxazol-4-ylmethoxy)-pyrazine-2-carboxylic acid, was converted, using 4-aminotetrahydropyran instead of aminomethylcyclopropane, to the title compound (SiO₂, heptane:ethyl acetate 1:1 to 0:1, 83.8 mg, 87%) which was obtained as a white solid. MS: m/e = 382.2 [M+H]⁺.

### Example 20

### 5-(3-Pyridin-2-yl-isoxazol-4-ylmethoxy)-pyrazine-2-carboxylic acid isopropylamide

As described for example 19f, 5-(3-pyridin-2-yl-isoxazol-4-ylmethoxy)-pyrazine-2-carboxylic acid (83.6 mg, 2.8 mmol) was converted, using isopropylamine instead of 4-aminotetrahydropyran, to the title compound (SiO₂, heptane:ethyl acetate 4:1 to 3:7, 65 mg, 68%) which was obtained as a white solid. MS: m/e = 340.2 [M+H]⁺.

### Example 21

### 5-(3-Pyridin-2-yl-isoxazol-4-ylmethoxy)-pyrazine-2-carboxylic acid cyclopropylamide

As described for example 19f, 5-(3-pyridin-2-yl-isoxazol-4-ylmethoxy)-pyrazine-2-carboxylic acid (84.7 mg, 2.8 mmol) was converted, using cyclopropylamine instead of 4-aminotetrahydropyran, to the title compound (SiO₂, heptane:ethyl acetate 7:3 to 2:8, 78 mg, 81%) which was obtained as a white solid. MS: m/e = 338.3 [M+H]⁺.

## Claims

1. A compound of formula I 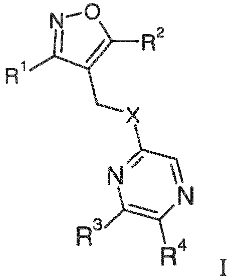 wherein
X is O or NH;
R¹ is phenyl or pyridine-2-yl, wherein the rings may be optionally substituted with one, two or three halo;
R² is hydrogen or C₁₋₄alkyl;
R³ and R⁴ each are independently
H,
-C(O)-R^{a}, wherein R^{a} is hydroxy, C₁₋₇alkoxy, C₁₋₇alkyl, phenoxy or phenyl,
-C(O)-NR^{b}R^{c}, wherein R^{b} and R^{c} are each independently
H,
C₁₋₇alkyl, optionally substituted with one or more halo, hydroxy, or cyano,
-(CH₂)_{z}-C₃₋₇cycloalkyl, optionally substituted by one or more B, and z is 0, 1, 2, 3 or 4,
-(CH₂)_{y}-heterocyclyl, wherein y is 0, 1, 2, 3 or 4, and wherein heterocyclyl is optionally substituted by one or more A
R^{b} and R^{c} together with the nitrogen to which they are bound form a heterocyclyl moiety , optionally substituted with one or more A, or
or R³ and R⁴ together form an annelated benzo ring, the benzo ring is optionally substituted by one or more E,
A is hydroxy, oxo, C₁₋₇alkyl, C₁₋₇alkoxy, C₁₋₇haloalkyl, C₁₋₇hydroxyalkyl, halo, or CN,
B is halo, hydroxy, CN, C₁₋₄alkyl, or C₁₋₄haloalkyl,
E is halo, CN, NO₂, hydroxy, C₁₋₇alkyl, C₁₋₇alkoxy, C₁₋₇haloalkyl, C₁₋₇hydroxyalkyl, C₁₋₇cyanoalkyl, C₁₋₇haloalkoxy, or C₃₋₇cycloalkyl,
or a pharmaceutically acceptable salt thereof;
wherein the term "heterocyclyl" or "heterocyclyl moiety" refers to a monovalent saturated or partially saturated 3- to 7-membered monocyclic or 9- to 10-membered bicyclic ring system wherein one, two, three or four ring carbon atoms have been replaced by N, O or S, and with the attachment point on the saturated or partially unsaturated ring of said ring system.

2. The compound according to claim 1, wherein
R³ is H, or
R³ and R⁴ together form an annelated benzo ring, the benzo ring is optionally substituted by one or more halo, CN, NO₂, hydroxy, C₁₋₇alkyl, C₁₋₇alkoxy, C₁₋₇haloalkyl, C₁₋₇hydroxyalkyl.

3. The compound according to claim 1, wherein
R⁴ is H,
- C(O)-R^{a}, wherein R^{a} is hydroxy, C₁₋₇alkoxy, C₁₋₇alkyl, phenoxy or phenyl,
- C(O)-NR^{b}R^{c}, wherein R^{b} and R^{c} are each independently
H,
C₁₋₇alkyl, optionally substituted with one or more halo, hydroxy, or cyano,
-(CH₂)_{z}-C₃₋₇cycloalkyl, optionally substituted by one or more B, and z is 0, 1, 2, 3 or 4,
-(CH₂)_{y}-heterocyclyl, wherein y is 0, 1, 2, 3 or 4, and wherein heterocyclyl is optionally substituted by one or more A
or R³ and R⁴ together form an annelated benzo ring, the benzo ring is optionally substituted by one or more E,
A is hydroxy, oxo, C₁₋₇alkyl, C₁₋₇alkoxy, C₁₋₇haloalkyl, C₁₋₇hydroxyalkyl, halo, or CN,
B is halo, hydroxy, CN, C₁₋₄alkyl, or C₁₋₄haloalkyl,
E is halo, CN, NO₂, hydroxy, C₁₋₇alkyl, C₁₋₇alkoxy, C₁₋₇haloalk_{Y}l, C₁₋₇hydroxyalkyl, C₁₋₇cyanoalkyl, C₁₋₇haloalkoxy, or C₃₋₇cycloalkyl;
wherein the term "heterocyclyl" or "heterocyclyl moiety" has the meaning as defined in claim 1.

4. A compound of formula I according to claim 1 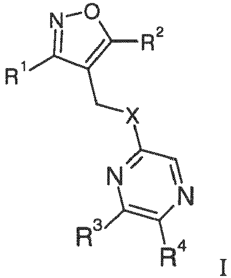 wherein
X is O or NH;
R¹ is phenyl or pyridine-2-yl, wherein the rings may be optionally substituted with halogen;
R² is H or C₁₋₄alkyl;
R³ and R⁴ each are independently
H,
-C(O)-R^{a}, wherein R^{a} is hydroxy, C₁₋₇alkoxy or C₁₋₇alkyl,
-C(O)-NR^{b}R^{c}, wherein R^{b} and R^{c} are each independently
hydrogen, C₁₋₇alkyl, optionally substituted with one or more halogen or hydroxy, or are independently from each other -(CH₂)_{z}-C₃₋₇cycloalkyl, optionally substituted by one or more halogen for z being 0 or 1,
or are independently from each other heterocyclyl,
or R³ and R⁴ together form an annelated benzo ring,
or a pharmaceutically acceptable salt thereof;
wherein the term "heterocyclyl" or "heterocyclyl moiety" has the meaning as defined in claim 1.

5. The compound according to any one of claims 1 to 4, wherein the compound is 5-(5-methyl-3-phenyl-isoxazol-4-ylmethoxy)-pyrazine-2-carboxylic acid methyl ester, 5-(5-methyl-3-phenyl-isoxazol-4-ylmethoxy)-pyrazine-2-carboxylic acid cyclopropylmethyl-amide, 5-(5-methyl-3-phenyl-isoxazol-4-ylmethoxy)-pyrazine-2-carboxylic acid isopropylamide, 5-(5-methyl-3-phenyl-isoxazol-4-ylmethoxy)-pyrazine-2-carboxylic acid *tert*-butylamide, 5-(5-methyl-3-phenyl-isoxazol-4-ylmethoxy)-pyrazine-2-carboxylic acid cyclopropylamide, 5-(5-methyl-3-phenyl-isoxazol-4-ylmethoxy)-pyrazine-2-carboxylic acid (tetrahydro-pyran-4-yl)-amide, 5-(5-methyl-3-phenyl-isoxazol-4-ylmethoxy)-pyrazine-2-carboxylic acid (4,4-difluoro-cyclohexyl)-amide, 2-(5-methyl-3-phenyl-isoxazol-4-ylmethoxy)-quinoxaline, 5-[(5-methyl-3-phenyl-isoxazol-4-ylmethyl)-amino]-pyrazine-2-carboxylic acid isopropylamide, 5-[(5-methyl-3-phenyl-isoxazol-4-ylmethyl)-amino]-pyrazine-2-carboxylic acid cyclopropylamide, 5-[(5-methyl-3-phenyl-isoxazol-4-ylmethyl)-amino]-pyrazine-2-carboxylic acid (tetrahydropyran-4-yl)-amide, 5-[3-(5-fluoro-pyridin-2-yl)-5-methyl-isoxazol-4-ylmethoxy]-pyrazine-2-carboxylic acid (tetrahydro-pyran-4-yl)-amide, 5-[3-(4-fluoro-phenyl)-isoxazol-4-ylmethoxy]-pyrazine-2-carboxylic acid (2,2,2-trifluoro-ethyl)-amide, 5-[3-(4-fluoro-phenyl)-isoxazol-4-ylmethoxy]-pyrazine-2-carboxylic acid cyclopropylmethyl-amide, 5-[3-(4-fluoro-phenyl)-isoxazol-4-ylmethoxy]-pyrazine-2-carboxylic acid isopropylamide, 5-[3-(4-chloro-phenyl)-isoxazol-4-ylmethoxy]-pyrazine-2-carboxylic acid isopropylamide, 5-[3-(4-chloro-phenyl)-isoxazol-4-ylmethoxy]-pyrazine-2-carboxylic acid ((S)-2-hydroxy-1-methyl-ethyl)-amide, 5-[3-(4-chloro-phenyl)-isoxazol-4-ylmethoxy]-pyrazine-2-carboxylic acid ((R)-2-hydroxy-1-methyl-ethyl)-amide, 5-(3-pyridin-2-yl-isoxazol-4-ylmethoxy)-pyrazine-2-carboxylic acid (tetrahydro-pyran-4-yl)-amide, 5-(3-pyridin-2-yl-isoxazol-4-ylmethoxy)-pyrazine-2-carboxylic acid isopropylamide or 5-(3-pyridin-2-yl-isoxazol-4-ylmethoxy)-pyrazine-2-carboxylic acid cyclopropylamide

6. A process to prepare the compound of formula I 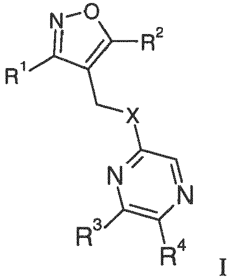 wherein X, and R¹ to R⁴ are as defined in claim 1, comprising the steps of
a) reacting a compound of formula VIII-a 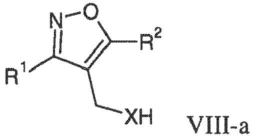 with a compound of formula IX 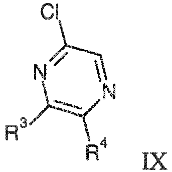 in the presence of a base, in a solvent.

7. A medicament containing at least one compound of formula I according to any one of claims 1 to 5.

8. The use of a compound of formula I according to any one of claims 1 to 5 for the preparation of a medicament useful as cognitive enhancer or for the preparation of a medicament for the treatment of cognitive disorders.

9. The use of a compound of formula I according to claim 8 for the preparation of a medicament for the treatment of Alzheimer's disease.

## Patentansprüche

1. Eine Verbindung der Formel I 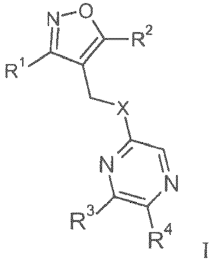 wobei
X O oder NH ist;
R¹ Phenyl oder Pyridin-2-yl ist, wobei die Ringe gegebenenfalls substituiert sein können mit einem, zwei oder drei Halogen;
R² Wasserstoff oder C₁₋₄-Alkyl ist;
R³ und R⁴ jeweils unabhängig
H,
-C(O)-R^{a}, wobei R^{a} Hydroxy, C₁₋₇-Alkoxy, C₁₋₇-Alkyl, Phenoxy oder Phenyl ist,
-C(O)-NR^{b}R^{c}, wobei R^{b} und R^{c} jeweils unabhängig
H,
C₁₋₇Alkyl, gegebenenfalls substituiert mit einem oder mehreren aus Halogen, Hydroxy, oder Cyano,
-(CH₂)_{z}-C₃₋₇-Cycloalkyl, gegebenenfalls substituiert mit einem oder mehreren B, und z gleich 0, 1, 2, 3 oder 4 ist,
-(CH₁)_{y}-Heterocyclyl, wobei y gleich 0, 1, 2, 3 oder 4 ist, und wobei Heterocyclyl gegebenenfalls substituiert ist mit einem oder mehreren A,
sind,
R^{b} und R^{c} zusammen mit dem Stickstoff, an den sie gebunden sind, eine Heterocyclyleinheit bilden, die gegebenenfalls substituiert ist mit einem oder mehreren A,
oder R³ und R⁴ zusammen einen annelierten Benzoring bilden, wobei der Benzoring gegebenenfalls substituiert ist mit einem oder mehreren E,
A Hydroxy, Oxo, C₁₋₇-Alkyl, C₁₋₇-Alkoxy, C₁₋₇-Halogenalkyl, C₁₋₇-Hydroxyalkyl, Halogen, oder CN ist,
B Halogen, Hydroxy, CN, C₁₋₄-Alkyl, oder C₁₋₄-Halogenalkyl ist,
E Halogen, CN, NO₂ Hydroxy, C₁₋₇-Alkyl, C₁₋₇-Alkoxy, C₁₋₇-Halogenalkyl, C₁₋₇-Hydroxyalkyl, C₁₋₇-Cyanoalkyl, C₁₋₇-Halogenalkoxy, oder C₃₋₇-Cycloalkyl ist,
oder ein pharmazeutisch verträgliches Salz davon;
wobei sich der Begriff "Heterocyclyl" oder "Heterocyclyleinheit" auf ein monovalentes gesättigtes oder teilweise gesättigtes 3- bis 7-gliedriges monozyklisches oder 9- bis 10-gliedriges bizyklisches Ringsystem bezieht, wobei ein, zwei, drei oder vier Ringkohlenstoffatome durch N, O oder S ersetzt wurden, und mit dem Anknüpfungspunkt an dem gesättigten oder teilweise ungesättigten Ring des Ringsystems.

2. Die Verbindung gemäß Anspruch 1, wobei
R³ H ist, oder
R³ und R⁴ zusammen einen annelierten Benzoring bilden, wobei der Benzoring gegebenenfalls substituiert ist mit einem oder mehreren aus Halogen, CN, NO₂, Hydroxy, C₁₋₇-Alkyl, C₁₋₇-Alkoxy, C₁₋₇-Halogenalkyl, C₁₋₇-Hydroxyalkyl.

3. Die Verbindung gemäß Anspruch 1, wobei
R⁴ H,
- C(O)-R^{a} wobei R^{a} Hydroxy, C₁₋₇-Alkoxy, C₁₋₇-Alkyl, Phenoxy oder Phenyl ist,
- C(O)-NR^{b}R^{c}, wobei R^{b} und R^{c} jeweils unabhängig
H,
C₁₋₇-Alkyl, gegebenenfalls substituiert mit einem oder mehreren aus Halogen, Hydroxy, oder Cyano,
-(CH₂)_{z}-C₃₋₇-Cycloalkyl, gegebenenfalls substituiert mit einem oder mehreren B, und z gleich 0, 1, 2, 3 oder 4 ist,
-(CH₂)_{y}-Heterocyclyl, wobei y gleich 0, 1, 2, 3 oder 4 ist, und wobei Heterocyclyl gegebenenfalls substituiert ist mit einem oder mehreren A,
sind,
ist,
oder R³ und R⁴ zusammen einen annelierten Benzoring bilden, wobei der Benzoring gegebenenfalls substituiert ist mit einem oder mehreren E,
A Hydroxy, Oxo, C₁₋₇-Alkyl, C₁₋₇-Alkoxy, C₁₋₇-Halogenalkyl, C₁₋₇-Hydroxyalkyl, Halogen, oder CN ist,
B Halogen, Hydroxy, CN, C₁₋₄-Alkyl, oder C₁₋₄-Halogenalkyl ist,
E Halogen, CN, NO₂, Hydroxy, C₁₋₇-Alkyl, C₁₋₇-Alkoxy, C₁₋₇-Halogenalkyl, C₁₋₇-Hydroxyalkyl, C₁₋₇-Cyanoalkyl, C₁₋₇-Halogenalkoxy, oder C₃₋₇Cycloalkyl ist;
wobei der Begriff "Hetercyclyl" oder "Heterocyclyleinheit" die Bedeutung wie in Anspruch 1 hat.

4. Eine Verbindung der Formel I gemäß Anspruch 1 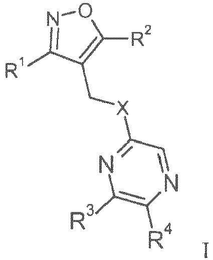 wobei
X O oder NH ist;
R¹ Phenyl oder Pyridin-2-yl ist, wobei die Ringe gegebenenfalls substituiert sein können mit Halogen;
R² H oder C₁₋₄-Alkyl ist;
R³ und R⁴ jeweils unabhängig
H,
-C(O)-R^{a}, wobei R^{a} Hydroxy, C₁₋₇-Alkoxy oder C₁₋₇-Alkyl ist,
-C(O)-NR^{b}R^{c}, wobei R^{b} und R^{c} jeweils unabhängig Wasserstoff, C₁₋₇-Alkyl, gegebenenfalls substituiert mit einem oder mehreren aus Halogen oder Hydroxy, sind oder unabhängig voneinander -(CH₂)_{z}-C₃₋₇-Cycloalkyl, gegebenenfalls substituiert mit einem oder mehreren aus Halogen, wobei z gleich 0 oder 1 ist, sind,
oder unabhängig voneinander Heterocyclyl sind,
oder R³ und R⁴ zusammen einen annelierten Benzoring bilden,
oder ein pharmazeutisch verträgliches Salz davon;
wobei der Begriff "Hetercyclyl" oder "Heterocyclyleinheit" die Bedeutung wie in Anspruch 1 hat.

5. Die Verbindung gemäß einem der Ansprüche 1 bis 4, wobei die Verbindung 5-(5-Methyl-3-phenyl-isoxazol-4-ylmethoxy)-pyrazin-2-carbonsäure-methylester, 5-(5-Methyl-3-phenyl-isoxazol-4-ylmethoxy)-pyrazin-2-carbonsäure-cyclopropylmethyl-amid, 5-(5-Methyl-3-phenyl-isoxazol-4-ylmethoxy)-pyrazin-2-carbonsäure-isopropylamid, 5-(5-Methyl-3-phenyl-isoxazol-4-ylmethoxy)-pyrazin-2-carbonsäure-tert-butylamid, 5-(5-Methyl-3-phenyl-isoxazol-4-ylmethoxy)-pyrazin-2-carbonsäure-cyclopropylamid, 5-(5-Methyl-3-phenyl-isoxazol-4-ylmethoxy)-pyrazin-2-carbonsäure-(tetrahydro-pyran-4-yl)-amid, 5-(5-Methyl-3-phenyl-isoxazol-4-ylmethoxy)-pyrazin-2-carbonsäure-(4,4-difluor-cyclohexyl)-amid, 2-(5-Methyl-3-phenyl-isoxazol-4-ylmethoxy)-chinoxalin, 5-[(5-Methyl-3-phenyl-isoxazol-4-ylmethyl)-amino]-pyrazin-2-carbonsäure-isopropylamid, 5-[(5-Methyl-3-phenyl-isoxazol-4-ylmethyl)-amino]-pyrazin-2-carbomsäure-cyclopropylamid, 5-[(5-Methyl-3-phenyl-isoxazol-4-ylmethyl)-amino]-pyrazin-2-carbonsäure-(tetrahydro-pyran-4-yl)-amid, 5-[3-(5-Fluor-pyridin-2-yl)-5-methyl-isoxazol-4-ylmethoxy]-pyrazin-2-carbonsäure-(tetrahydro-pyran-4-yl)-amid, 5-[3-(4-Fluor-phenyl)-isoxazol-4-ylmethoxy]-pyrazin-2-carbonsäure-(2,2,2-trifluor-ethyl)-amid, 5-[3-(4-Fluor-phenyl)-isoxazol-4-ylmethoxy]-pyrazin-2-carbonsäure-cyclopropylmethyl-amid, 5-[3-(4-Fluor-phenyl)-isoxazol-4-ylmethoxy]-pyrazin-2-carbonsäure-isopropylamid, 5-[3-(4-Chlor-phenyl)-isoxazol-4-ylmethoxy]-pyrazin-2-carbonsäure-isopropylamid, 5-[3-(4-Chlor-phenyl)-isoxazol-4-ylmethoxy]-pyrazin-2-carbonsäure-((S)-2-hydroxy-1-methyl-ethyl)-amid, 5-[3-(4-Chlor-phenyl)-isoxazol-4-ylmethoxy]-pyrazin-2-carbonsäure-((R)-2-hydroxy-1-methyl-ethyl)-amid, 5-(3-Pyridin-2-yl-isoxazol-4-ylmethoxy)-pyrazin-2-carbonsäure-(tetrahydro-pyran-4-yl)-amid, 5-(3-Pyridin-2-yl-isoxazol-4-ylmethoxy)-pyrazin-2-carbonsäure-isopropylamid oder 5-(3-Pyridin-2-yl-isoxazol-4-ylmethoxy)-pyrazin-2-carbonsäure-cyclopropylamid ist.

6. Ein Verfahren zur Herstellung der Verbindung der Formel I 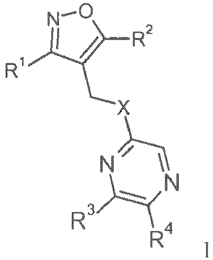 wobei X, und R¹ bis R⁴ wie in Anspruch 1 definiert sind, umfassend die Schritte
a) Umsetzen einer Verbindung der Formel VIII-a 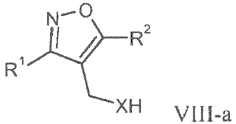 mit einer Verbindung der Formel IX 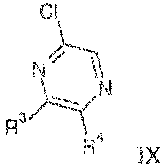 in Gegenwart einer Base, in einem Lösungsmittel.

7. Ein Medikament, das mindestens eine Verbindung der Formel I gemäß einem der Ansprüche 1 bis 5 enthält.

8. Die Verwendung einer Verbindung der Formel I gemäß einem der Ansprüche 1 bis 5 zur Herstellung eines Medikaments, das als kognitiver Enhancer geeignet ist, oder zur Herstellung eines Medikaments zur Behandlung von kognitiven Störungen.

9. Die Verwendung einer Verbindung der Formel I gemäß Anspruch 8 zur Herstellung eines Medikaments zur Behandlung von Alzheimer-Krankheit.

## Revendications

1. Composé de formule I 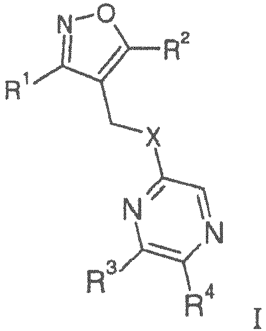 dans laquelle
X es tO ou NH;
R¹ est un groupe phényle ou pyridin-2-yle, dans lequel les cycles peuvent être éventuellement substitués par un, deux ou trois groupes halogéno ;
R² est un atome d'hydrogène ou un groupe alkyle en C, à C₄ ;
R³ et R⁴ sont chacun indépendamment
H,
-C(O)-R^{a}, dans lequel R^{a} est un groupe hydroxy, alcoxy en C, à C₇, alkyle en C, à C₇, phénoxy ou phényle,
-C(O)-NR^{b}R^{c}, dans lequel R^{b} et R^{c} sont chacun indépendamment H,
un groupe alkyle en C, à C₇, éventuellement substitué par un ou plusieurs groupes halogéno, hydroxy ou cyano, -(CH₂)_{z}-cycloalkyle en C₃ à C₇, éventuellement substitué par un ou plusieurs B,
et z est 0, 1, 2, 3 ou 4,
- (CH₂)_{y}-hétérocyclyle, dans lequel y est 0, 1, 2, 3 ou 4, et dans lequel le groupe hétérocyclyle est éventuellement substitué par un ou plusieurs A,
R^{b} et R^{c} conjointement avec l'atome d'azote auquel ils sont liés forment une fraction hétérocyclyle, éventuellement substituée par un ou plusieurs A, ou
R³ et R^{a} forment conjointement un cycle benzo annelé, le cycle benzo étant éventuellement substitué par un ou plusieurs E,
A est un groupe hydroxy, oxo, alkyle en C, à C₇, alcoxy en C₁ à C₇, halogénoalkyle en C, à C₇, hydroxyalkyle en C, à C₇, halogéno ou CN,
B est un groupe halogéno, hydroxy, CN, alkyle en C, à C₄ ou halogénoalkyle en C, à C₄,
E est un groupe halogéno, CN, NO₂, hydroxy, alkyle en C, à C₇, alcoxy en C, à C₇, halogénoalkyle en C, à C₇, hydroxyalkyle en C, à C₇, cyanoalkyle en C, à C₇, halogénoalcoxy en C, à C₇ ou cycloalkyle en C₃ à C₇,
ou un sel pharmaceutiquement acceptable de ceux-ci ;
dans lequel le terme « hétérocyclyle » ou « fraction hétérocyclyle » désigne un système cyclique monovalent, saturé ou partiellement saturé monocyclique de 3 à 7 chaînons ou bicyclique de 9 à 10 chaînons, dans lequel un, deux, trois ou quatre atomes de carbone cyclique ont été remplacés par N, O ou S, et avec le point de fixation sur le cycle saturé ou partiellement insaturé dudit système cyclique.

2. Composé selon la revendication 1, dans lequel
R³ est H, ou
R³ et R⁴ forment conjointement un cycle benzo annelé, le cycle benzo étant éventuellement substitué par un ou plusieurs groupes halogéno, CN, NO₂, hydroxy, alkyle en C, à C₇, alcoxy en C, à C₇, halogénoalkyle en C₁ à C₇, hydroxyalkyle en C, à C₇.

3. Composé selon la revendication 1, dans lequel
R⁴ est H
-C(O)-R^{a}, dans lequel R^{a} est un groupe hydroxy, alcoxy en C₁ à C₇, alkyle en C, à C₇, phénoxy ou phényle,
-C(O)-NR^{b}R^{c}, dans lequel R^{b} et R^{c} sont chacun indépendamment H,
un groupe alkyle en C, à C₇, éventuellement substitué par un ou plusieurs groupes halogéno, hydroxy, ou cyano, -(CH₂)_{z}-cycloalkyle en C₃ à C₇, éventuellement subtsitué par un ou plusieurs b,
et z est 0, 1, 2, 3 ou 4,
-(CH₂)_{y}-hétérocyclyle, dans lequel y est 0, 1, 2, 3 ou 4, et dans lequel le groupe hétérocyclyle est éventuellement substitué par un ou plusieurs A,
ou R³ et R⁴ forment conjointement un cycle benzo annelé, le cycle benzo étant éventuellement substitué par un ou plusieurs E,
A est un groupe hydroxy, oxo, alkyle en C, à C₇, alcoxy en C, à C₇, halogénoalkyle en C, à C₇, hydroxyalkyle en C₁ à C₇, halogéno ou CN,
B est un groupe halogéno, hydroxy, CN, alkyle en C₁ à C₄ ou halogénoalkyle en C, à C₄,
E est un groupe halogéno, CN, NO₂, hydroxy, alkyle en C₁ à C₇, alcoxy en C, à C₇, halogénoalkyle en C₁ à C₇, hydroxyalkyle en C, à C₇, cyanoalkyle en C, à C₇, halogénoalcoxy en C, à C₇ ou cycloalkyle en C₃ à C₇,
dans lequel le terme « hétérocyclyle » ou « fraction hétérocyclyle » a la signification indiquée dans la revendication 1.

4. Composé de formule I selon la revendication 1 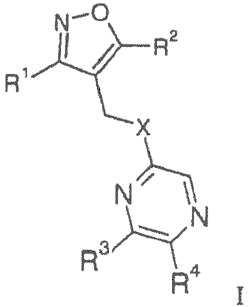 dans laquelle
X est O ou NH ;
R¹ est un groupe phényle ou pyridin-2-yle, dans lequel les cycles peuvent être éventuellement substitués par un atome d'halogène ;
R² est un atome de H ou un groupe alkyle en C₁ à C₄ ;
R³ et R⁴ sont chacun indépendamment
H,
-C(O)-R^{a}, dans lequel R^{a} est un groupe hydroxy, alcoxy en C, à C₇ ou alkyle en C, à C₇,
-C(O)-NR^{b}R^{c}, dans lequel R^{b} et R^{c} sont chacun indépendamment un atome d'hydrogène, un groupe alkyle en C, à C₇, éventuellement substitué par un ou plusieurs groupes halogéno ou hydroxy ou sont indépendamment l'un de l'autre
-(CH₂)_{z}-cycloalkyle en C₃ à C₇, éventuellement substitué par un ou plusieurs atomes d'halogène pour z qui est 0 ou 1,
ou sont indépendamment l'un de l'autre, un groupe hétérocyclyle,
ou R³ et R⁴ forment conjointement un cycle benzo annelé,
ou un sel pharmaceutiquement acceptable de ceux-ci ;
dans lequel le terme « hétérocyclyle » ou « fraction hétérocyclyle » a la signification indiquée dans la revendication 1.

5. Composé selon l'une quelconque des revendications 1 à 4, dans lequel le composé est
l'ester méthylique de l'acide 5-(5-méthyl-3-phényl-isoxazol-4-ylméthoxy)-pyrazine-2-carboxylique,
le cyclopropylméthylamide de l'acide 5-(5-méthyl-3-phényl-isoxazol-4-ylméthoxy)-pyrazine-2-carboxylique,
l'isopropylamide de l'acide 5-(5-méthyl-3-phényl-isoxazol-4-ylméthoxy)-pyrazine-2-carboxylique,
le tert-butylamide de l'acide 5-(5-méthyl-3-phényl-isoxazol-4-ylméthoxy)-pyrazine-2-carboxylique,
le cyclopropylamide de l'acide 5-(5-méthyl-3-phényl-isoxazol-4-ylméthoxy)-pyrazine-2-carboxylique,
le (tétrahydro-pyran-4-yl)-amide de l'acide 5-(5-méthyl-3-phényl-isoxazol-4-ylméthoxy)-pyrazine-2-carboxylique,
le (4,4-difluoro-cyclohexyl)-amide de l'acide 5-(5-méthyl-3-phényl-isoxazol-4-ylméthoxy)-pyrazine-2-carboxylique,
la 2-(5-méthyl-3-phényl-isoxazol-4-ylméthoxy)-quinoxaline.
l'isopropylamide de l'acide 5-[(5-méthyl-3-phényl-isoxazol-4-ylméthyl)-amino]-pyrazine-2-carboxylique,
le cyclopropylamide de l'acide 5-[(5-méthyl-3-phényl-isoxazol-4-ylméthyl)-amino]-pyrazine-2-carboxylique,
le (tétrahydro-pyran-4-yl)-amide de l'acide 5-[(5-méthyl-3-phényl-isoxazol-4-ylméthyl)-amino]-pyrazine-2-carboxylique,
le (tétrahydro-pyran-4-yl)-amide de l'acide 5-[3-(5-fluoro-pyridin-2-yl)-5-méthyl-isoxazol-4-ylméthoxy]-pyrazine-2-carboxylique,
le (2,2,2-trifluoro-éthyl)-amide de l'acide 5-[3-(4-fluoro-phényl)-isoxazol-4-ylméthoxy]-pyrazine-2-carboxylique,
le cyclopropylméthylamide de l'acide 5-[3-(4-fluoro-phényl)-isoxazol-4-ylméthoxy]-pyrazine-2-carboxylique,
l'isopropylamide de l'acide 5-[3-(4-fluoro-phényl)-isoxazol-4-ylméthoxy]-pyrazine-2-carboxylique,
l'isopropylamide de l'acide 5-[3-(4-chloro-phényl)-isoxazol-4-ylméthoxy]-pyrazine-2-carboxylique,
le ((S)-2-hydroxy-1-méthyl-éthyl)-amide de l'acide 5-[3-(4-chloro-phényl)-isoxazol-4-ylméthoxy]-pyrazine-2-carboxylique,
le ((R)-2-hydroxy-1-méthyl-éthyl)-amide de l'acide 5-[3-(4-chloro-phényl)-isoxazol-4-ylméthoxy]-pyrazine-2-carboxylique,
le (tétrahydro-pyran-4-yl)-amide de l'acide 5-(3-pyridin-2-yl-isoxazol-4-ylméthoxy)-pyrazine-2-carboxylique,
l'isopropylamide de l'acide 5-(3-pyridin-2-yl-isoxazol-4-ylméthoxy)-pyrazine-2-carboxylique, ou
le cyclopropylamide de l'acide 5-(3-pyridin-2-yl-isoxazol-4-ylméthoxy)-pyrazine-2-carboxylique,

6. Procédé de préparation du composé de formule I 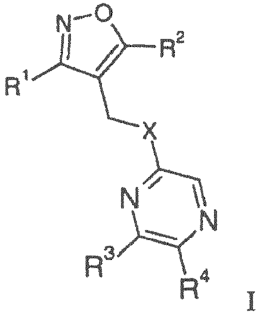 dans laquelle X, et R¹ à R⁴ sont tels que définis dans la revendication 1, comprenant les étapes de
a) réaction d'un composé de formule VIII-a 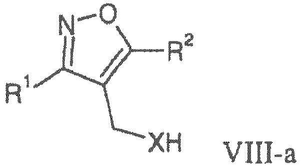 avec un composé de formule IX 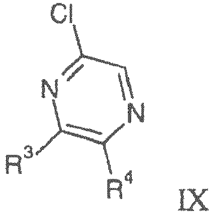 en présence d'une base, dans un solvant.

7. Médicament contenant au moins un composé de formule I selon l'une quelconque des revendications 1 à 5.

8. Utilisation d'un composé de formule I selon l'une quelconque des revendications 1 à 5 pour la préparation d'un médicament utile comme agent d'amélioration cognitive ou pour la préparation d'un médicament pour le traitement de troubles cognitifs.

9. Utilisation d'un composé de formule I selon la revendication 8 pour la préparation d'un médicament pour le traitement de la maladie d'Alzheimer.
